# DEMANDE DE BREVET EUROPEEN

(11) **EP 2 314 290 A1**
(43) Date de publication de la demande: **27.04.2011**
(21) Numéro de dépôt: 10195688.6
(22) Date de dépôt: 01.06.2004
(51) Int. Cl.: A61K 31/137, A61K 31/138, A61K 31/165, A61K 31/198, A61K 31/295, A61K 33/26, A61K 31/4458, A61K 45/06, A61P 25/00

(54) **Utilisation du fer pour le traitement du trouble du déficit de l'attention/hyperactivité chez les enfants**

(30) Priorité: 30.05.2003 FR 0306581
(62) Demande divisionnaire de: 04767222.5
(71) Demandeur: Assistance Publique - Hôpitaux de Paris, 75004 Paris (FR)
(72) Inventeur: Konofal, Eric, 60300, SENLIS (FR)
(74) Mandataire: Warcoin, Jacques

(57) **Abrégé**

L'invention concerne l'utilisation du fer pour la préparation d'un médicament destiné au traitement préventif et/ou curatif du trouble du déficit de l'attention/hyperactivité (TDHA) ou d'au moins un de ces symptômes, chez un patient nécessitant un tel traitement.

## Description

La présente invention concerne le domaine de la santé humaine et plus particulièrement le traitement du trouble "déficit de l'attention/hyper-activité". Plus particulièrement, la présente invention concerne l'utilisation du fer ou l'un de ses sels pharmaceutiquement acceptables, seul ou en association avec un ou plusieurs composés psycho-stimulants, pour la préparation d'un médicament destiné au traitement de la TDAH et des symptômes associés.

Le trouble "déficit de l**'**attention/hyper-activité" de l'enfant (TDAH) est un trouble comportemental qui constitue le premier motif de consultation en psychologie de l'enfant et de l'adolescent. Ce syndrome très répandu affecte 6 à 10 % des enfants d'âge scolaire.

Sur le plan clinique, ce trouble associe une inattention, une impulsivité et une hyperactivité motrice inadaptée à l'environnement de l'enfant. Mal organisés et étourdis, ces enfants finissent parfois par ne plus suivre en classe. L'agitation motrice excessive, incompatible avec les relations sociales et pouvant parfois même conduire à une déscolarisation prématurée, est probablement le symptôme qui amènera les parents à consulter un spécialiste.

La physiopathologie de ce trouble reste encore aujourd'hui discutée bien que, pour un bon nombre d'auteurs, l'hypothèse d'une implication des systèmes dopaminergiques et noradrénergiques semble validée [Spencer et al., Pharmacotherapy of attention deficit hyperactivity disorder. Child Adolesc Psychiatr Clin N Am. 2000; 9(1):77-97] . Ce dysfonctionnement de la neurotransmission dopaminergique semble être impliquée dans les symptômes d'hyperactivité motrice excessive caractéristique du TDAH de l'enfant. De fait, l'amélioration de l'hyperactivité motrice par les psychostimulants dopaminergiques est souvent très significative mais néanmoins insuffisante.

Les insomnies, les difficultés d'endormissement, les réveils au cours de la nuit, éventuellement dus à une agitation motrice nocturne excessive, ainsi que les troubles attentionnels, tels que l'inattention, l'impatience et l'impulsivité semblent échapper à toute forme de traitement [Chervin et al., Associations between symptoms of inattention, hyperactivity, restless legs, and periodic leg movements. Sleep 2002 15;25(2):213-8; Gruber et al., instability of sleep patterns in children with attention-deficit/hyperactivity disorder. J Am Acad Child Adolesc Psychiatry. 2000;39(4):495- 501].

Il existe donc un réel besoin de développer de nouveaux traitements du TDAH qui permettent d'obtenir des résultats supérieurs à ceux obtenus avec les traitements actuels à base de psycho-stimulants et notamment de pouvoir traiter les symptômes qui échappent aux traitements actuels. C'est le but de la présente invention.

De manière tout à fait fortuite, l'inventeur a maintenant observé une ferritinémie anormalement basse chez les enfants atteints de TDAH. L'inventeur a en outre démontré une corrélation entre la sévérité des symptômes et la férritinémie. La présente invention se propose donc de fournir un traitement préventif et curatif du TDAH par la correction de l'hypoferritinémie observée chez ces patients.

Par le passé, différentes études ont été menées qui ont conduit à étudier l'implication du fer dans le TDAH. A l'origine de ces études est la constatation que des enfants atteints de TDAH ont une concentration basse en certains oligoéléments, dont le fer (Pour revue voir : Brue and Oakland, 2002 Alternatives Thérapies 8 :68-73) . Ainsi, en 1994, Kozielec et al. (Psychiatr.pol. 28 : 345-353) ont recherché un déficit en oligoéléments (magnésium, zinc, cuivre, calcium, fer) dans le TDAH. Les auteurs n'ont pas fait de lien entre le fer et le TDAH, et à l'inverse, la magnésémie retrouvée chez les enfants étudiés suggère aux auteurs d'évoquer l'intérêt d'une supplémentation en magnésium dans le TDAH. L'existence d'une motricité nocturne excessive a conduit Sever et al. (1997, Neuropsychobiology 35 :178-180) à étudier l'implication du fer dans le TDAH d'enfants non anémiés. Cette étude, qui reste la seule étude s'étant intéressée à montrer l'intérêt de traiter empiriquement des enfants présentant un TDAH, n'évoque pas dans ses résultats ni dans ses perspectives le rôle de la ferritine dans la physiopathologie du trouble. Les résultats obtenus ne montrent pas de rapport entre la sévérité des symptômes et la carence martiale. Les auteurs ont d'ailleurs conclu que le déficit en fer ne joue pas un rôle dans la physiopathologie du TDAH et qu'un traitement oral par le fer des enfants affectés de TDAH n'est pas recommandé. Finalement, ces résultats inconsistants et parfois contradictoires obtenus par les chercheurs et les médecins les ont conduit à privilégier d'autres approches thérapeutiques du TDAH qui ont cours actuellement, à base notamment de psycho-stimulants dopaminergiques.

La présente invention a donc pour objet l'utilisation du fer, ou l'un de ses sels pharmaceutiquement acceptables, pour la préparation d'un médicament destiné au traitement préventif et/ou curatif du trouble du déficit de l'attention/hyperactivité (TDAH) ou d'au moins un de ces symptômes chez un patient nécessitant un tel traitement. La ferritine est une protéine de stockage du fer (Connor et al., Pediatric Neurology 25 : p123- 124).

Dans le cadre de la présente invention, le diagnostic de Trouble Déficit de l'Attention/Hyperactivité (TDAH) est fondé selon les caractéristiques cliniques définies par la classification internationale, le Manuel Diagnostique et Statistique des troubles mentaux, DSM-IV (Diagnostic and Statistical Manual of mental disorders, 4ème ed., 1994) .

Les critères du DSM-IV incluent trois dimensions (inattention, impulsivité et hyperactivité), une efficience intellectuelle normale (QI>80), mais n'incluent aucune pathologie organique, ni neurologique.

Dans le cas de la présente invention, le patient est donc un enfant avec un QI>80, d'âge compris entre 5 et 12 ans, et présentant une carence martiale isolée mais non anémié, c'est-à-dire présentant un taux d'hémoglobine normal. Par l'expression "carence martiale", on entend une hypoferritinémie sans modification significative de la concentration sérique en récepteurs solubles de la transferrine.

Au sens de la présente invention, on entend par « fer », le fer sous la forme d'un atome de fer, de sel de fer, ou de fer organique, ou de toute formulation contenant du fer qui soit pharmaceutiquement acceptable. A titre de liste non exhaustive, le sel de fer pharmaceutiquement acceptable est sélectionné parmi les sels ferreux et les sels férriques, de préférence parmi l'ammonium citrate ferrique, le pyrophosphate ferrique, le ferrocholinate, l'ascorbate ferreux, l'aspartate ferreux, le chlorure ferreux, le sulfate ferreux, le tartrate ferreux, le fumarate ferreux, le gluconate ferreux, le gluceptate ferreux, le sulfate de glycine ferreux, le lactate ferreux, l'oxalate ferreux, le succinate ferreux. Selon un mode préféré de l'invention, le sel de fer est le sulfate ferreux, et de préférence du sulfate ferreux gastro-protégé telle la spécialité "Tardyféron" des Laboratoires Pierre Fabre Médicament. Alternativement, le fer pharmaceutiquement acceptable est sous la forme de fer dextran, de fer sucrose, de fer polymaltose, de fer sorbitol. Lorsque le fer est sous la forme de fer organique pharmaceutiquement acceptable, il s'agit de préférence de biglycinate de fer, de glycinate de fer ou de fer protéine succinylate.

La nature du sel administré au patient dépend de la voie d'administration retenue qui pourra être indifféremment la voie orale, anale, parentérale, intraveineuse, intramusculaire. De préférence, il s'agit de la voie orale.

Par "symptômes du TDAH", on entend désigner notamment les troubles attentionnels tels l'inattention, l'impulsivité, l'impatience, les troubles oppositionnels, mais également l'hyperactivité motrice diurne ou nocturne, et les insomnies. Par insomnie on entend désigner :
a. l'insomnie par endormissement qui se caractérise par des difficultés à s'endormir ;
b. l'insomnie de maintenance qui se caractérise par une hyperactivité motrice nocturne et des réveils en cours de nuit, et ;
c. l'insomnie psychopathologique généralement chronique et généralement liée à une anxiété, au stress et à des épisodes dépressifs.

Selon un mode préféré de réalisation, l'utilisation du fer ou de l'un de ses sels pharmaceutiquement acceptables selon l'invention est réalisée en association avec au moins un composé sélectionné parmi les psycho-stimulants, comme produit de combinaison pour une utilisation simultanée, séparée ou échelonnée dans le temps.

Par composés psycho-stimulants, on entend désigner les inhibiteurs de la recapture de la dopamine et/ou de la noradrénaline. Parmi ceux-ci, il convient de citer à titre non exhaustif la L-Dopa, la Dopamine, les agonistes de la L-dopa, les agonistes de la dopamine, la rotaline. Plus particulièrement, les composés psychostimulants sont choisis parmi le methylphénidate (spécialité Ritaline), le modafinil, l'atomoxétine, et les amphétamines, telles que la d-amphétamine, la déxédrine, la dexamphétamine.

La présente invention concerne également l'utilisation du fer, ou l'un de ses sels pharmaceutiquement acceptables en association avec au moins un composé sélectionné parmi les psycho-stimulants, notamment les inhibiteurs de la recapture de la dopamine et/ou de la noradrénaline, comme produit de combinaison pour une utilisation simultanée, séparée ou échelonnée dans le temps, pour la préparation d'un médicament destiné au traitement préventif et/ou curatif d'une pathologie sélectionnée parmi le TDAH ou au moins un des symptômes du TDAH tels que l'hyperactivité motrice nocturne et/ou diurne. La composition pharmaceutique comprenant cette association et des excipients pharmaceutiquement acceptables fait également partie de l'invention.

Dans le cadre de la présente invention, la posologie martiale correspond à une prise journalière de sulfate ferreux comprise entre 0.1 mg et 10 g et de préférence comprise entre 10 mg et 2 g par jour, et plus particulièrement, d'au moins 50 mg, au moins 150 mg, d'au moins 200 mg, d'au moins 250 mg, d'au moins 300 mg, d'au moins 350 mg, d'au moins 400 mg, d'au moins 450 mg, d'au moins 500 mg, d'au moins 550 mg, d'au moins 600 mg, d'au moins 700 mg, d'au moins 800 mg, d'au moins 900 mg, d'au moins 1 g par jour, de préférence comprise entre 400 mg et 750 mg par jour, de préférence environ 500 mg, en une ou plusieurs prises quotidienne.

Le patient selon l'invention est choisi parmi un nouveau-né, un enfant, un adolescent, un adulte. Selon un mode préféré de réalisation, il s'agit d'un enfant d'âge environ 5 à 12 ans, et/ou d'un adolescent. Le patient selon l'invention est affecté d'une carence martiale mais est non anémié, c'est-à-dire qu'il ne présente pas de baisse du taux d'hémoglobine. Par l'expression "carence martiale", on entend une hypoferritinémie sans modification significative de la concentration sérique en récepteurs solubles de la transferrine. La carence en ferritine peut être mesurée dans le sérum, mais également dans tout autres liquides biologiques tels que le liquide céphalo-rachidien.

Une carence en ferritine correspond à une concentration sérique en ferritine du patient adulte inférieure à environ 50 pg/litre. Cette hypoférritinémie peut atteindre des concentrations en ferritine inférieures à environ 40 µg/1, voire inférieures à environ 35 µg/1, inférieures à environ 30 µg/1, inférieures à environ 20 µg/1, inférieures à environ 15 µg/1, voire même inférieures à environ 10 µg/1. Les techniques de dosage de la ferritine sérique sont bien connues de l'homme de l'art. On peut citer la méthode immunoenzymatique (Kit IMX ferritine, Abott Laboratories).

Le patient selon l'invention présente en outre une concentration sérique normale de récepteurs solubles à la transferrine. La transferrine est impliquée dans l'acquisition du fer par les cellules de l'organisme ; cette acquisition est contrôlée par le nombre de récepteurs à la transferrine existant à la surface cellulaire. La concentration de ces récepteurs peut être évalués par des techniques connues de l'homme de l'art telles que la néphélémétrie (Ruivard et al., 2000 Rev. Méd. Interne 21 : 837-843). Une fourchette de concentration normale des récepteurs solubles à la transferrine est de 2,0-4,50 mg/l pour les hommes et de 1,80-4,70 mg/l pour les femmes (voir Kit RsTF Ref.2148315 de Roche).

Le rôle du fer au niveau du système nerveux central est souvent rapporté en neurophysiopathologie fondamentale comme clinique. Une asthénie fonctionnelle, intellectuelle, un syndrome de fatigue chronique, ou à l'inverse une instabilité psychomotrice et une irritabilité peuvent être la conséquence d'une carence martiale (Lozoff, 1989 Adv Pediatr 1989; 6: 331-59). Le rôle du fer dans la physiopathologie de maladies neurologiques, et notamment dans la Maladie de Parkinson Idiopathique est connu depuis plus de trente ans. L'évidence d'une augmentation martiale notamment dans certaines structures cérébrales (p.e. noyau denté) dans des pathologies neurodégénératives rares (p.e. ataxie de Friedreich) est également connue. Plus récemment, le rôle des récepteurs de la transferrine dans certains processus neurophysiopathologiques vient d'être documenté (Marder K, et al. 1998 Neurology 50, 4:1138-40). Une augmentation en nombre des récepteurs de la transferrine des cellules de l'endothélium des capillaires cérébraux pourrait être responsable de l'accumulation cytoplasmique du fer dans les cellules des neurones des ganglions de la base (globus pallidus, substantia nigra, noyau rouge, et noyau denté). Un dysfonctionnement des récepteurs de la transferrine par hyperplasie (augmentation du nombre des récepteurs) au niveau central expliquerait l'accumulation du fer dans certaines structures impliquées dans les phénomènes de neurodégénérescence. A contrario, une diminution de ces récepteurs contribuerait à protéger les noyaux centraux du phénomène. Dans l'hypothèse d'une diminution de la ferritine plasmatique dans la physiopathologie du TDAH, une augmentation physiologique des récepteurs de la transferrine devrait se produire, comme elle se produit normalement en cas d'anémie, afin de ne pas mettre les structures cérébrales en carence martiale. Par contre, une absence de réponse (absence d'augmentation du nombre des récepteurs de la transferrine) conduirait une diminution martiale cérébrale et serait compatible avec un dysfonctionnement dopaminergique par baisse de sa synthèse et/ou de la stimulation des récepteurs dopaminergiques. La présente invention concerne donc également l'utilisation du fer ou l'un de ses sels pharmaceutiquement acceptables pour le traitement préventif de patient nouveau-né, enfant, adolescent, jeune adulte amené à développer à l'âge adulte une pathologie neuro-dégénérative caractérisée en ce que le dit patient nouveau-né, enfant, adolescent, jeune adulte présente au moins les symptômes suivants .
- une carence en ferritine, de sorte que la concentration sérique en ferritine est inférieure à 50 *ggn,*
- une concentration sérique normale des récepteurs solubles à transferrine,
- un trouble du déficit de l'attention/hyperactivité, ou au moins un de ces symptômes.

De préférence ledit patient est un enfant avec un QI>80, d'âge compris entre environ 5 et 12 ans et non anémié.

De préférence la dite pathologie neurodégénérative est la maladie de Parkinson, les ataxies cérébelleuses, l'ataxie de Friedrich, la maladie d'alzheimer, la chorée de Huntington, la sclérose latérale amyotrophique. Plus particulièrement, il s'agit de la maladie de Parkinson.

Egalement, la présente invention concerne également tout procédé ou kit de diagnostic utilisant la ferritine et/ou le fer et les récepteurs solubles à la transferrine comme marqueur de sévérité du TDAH. C'est la raison pour laquelle, l'invention fournit également un procédé in vitro de pronostic et/ou diagnostic du trouble du déficit de l'attention/ hyperactivité comprenant l'étape d'évaluer quantitativement chez un patient suspecté d'être affecté par le dit trouble, la concentration sérique de ferritine et la concentration en récepteur soluble de la transferrine, de sorte qu'une concentration en ferritine sérique inférieure à 50 µg/l et une concentration normale physiologique des récepteurs solubles à la transferrine indique que le patient est ou sera affecté du dit trouble. Le kit de diagnostic correspondant est également l'objet de la présente invention.

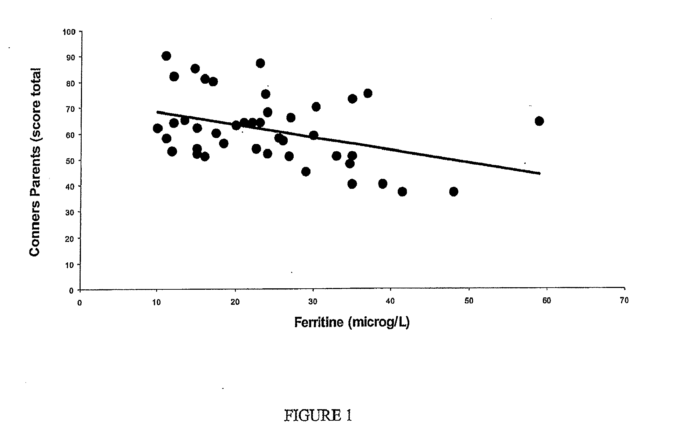

Enfin l'invention vise à protéger une source de fer seule ou en association avec au moins un psychostimulant, de préférence la ritaline, comme médicament ou comme principes actifs d'une composition pharmaceutique comprenant des excipients pharmaceutiquement acceptables, pour le traitement préventif et/ou curatif du TDAH ou l'un de ses symptômes. D'autres caractéristiques, buts et avantages de l'invention ressortiront des exemples qui suivent. L'invention ne se trouve pas limitée aux exemples particuliers mentionnés à simple titre illustratif et qui doivent être lus en regard de la figure suivante :
Figure 1 :
   Les valeurs de ferritine (normale = 34µg/L) sont inversement corrélées (p<0.01) à la sévérité des symptômes du TDAH exprimée par le Conners Parents (normale < 50).

### EXEMPLES

### 1. Méthode

Quarante-trois enfants, 36 garçons et 7 filles, d'un âge moyen de 9,2 ± 2,2 ans, ont participé à cette étude prospective. Leurs caractéristiques cliniques correspondaient aux critères du TDAH du Manuel diagnostique et statistique des troubles mentaux (Diagnostic and Statistical Manual of Mental Disorders) (4^{ème} édition, APA, 1994). Ces TDAH ont été confirmés par un entretien structuré (anamnèse). Les enfants ne souffraient d'aucune déficience physique, ni de malnutrition, ni de maladies mentales (QI>80) ou organiques et n'étaient sous aucun traitement incluant une supplémentation en fer ou des psycho-stimulants, ceci pour une durée d'au moins 2 mois avant l'étude.

La gravité des symptômes a été évaluée à l'aide de l'échelle d'évaluation de Conners (questionnaire à l'usage des parents), y compris pour les sous-échelles d'évaluation des capacités cognitives et du trouble oppositionnel. La ferritinémie a été mesurée, ainsi que le taux d'hémoglobine, l'hématocrite et le fer sérique par les méthodes classiques (Tests Elecsys, immuno-enzymologie). Les valeurs moyennes de la ferritine ont été classées en trois groupes : normal (> 34 µg/L), sub-normale (> 15 µg/L) et anormal (< 15 µg/L) .

### 2. Résultats

Le résultat moyen de tous les patients (61±13) soumis au questionnaire de Conners à l'usage des parents a révélé de graves TDAH. Ce résultat a été identique chez les filles (63+8) et chez les garçons (60+14). Le fer sérique (moyenne du groupe : 84±36 µg/100 ml), les taux d'hémoglobine et d'hématocrite étaient normales. La valeur moyenne de la ferritinémie était basse (25±12 µg/L) et notamment pathologique chez 33 sur 43 enfants présentant un TDAH (77%). Elle était identique chez les garçons (26±13 µg/L) et chez les filles (19±8 µg/L) et il n'y avait pas de relation avec l'âge.

Les relations entre les caractéristiques cliniques et biologiques des patients, en fonction de leur ferritinémie (anormale, sub-normale et normale) sont reportées dans le tableau 1. Les enfants ayant une ferritine anormale présentaient des symptômes cliniques plus graves pour le TDAH que les enfants ayant une ferritine normale (p< 0.01).

D'autre part, il y avait une corrélation négative entre le questionnaire de Conners à usage des parents et les valeurs de la ferritine (r= -0,41 p= 0,01). Il n'y avait pas de corrélation entre les scores du questionnaire de Conners à usage des parents pour les symptômes d'hyperactivité, de trouble cognitif et de trouble oppositionnel, et les valeurs de la ferritine.

**Tableau 1: Caractéristiques cliniques et biologiques dans les différents groupes d'enfants présentant TDAH.**

| | Valeurs moyennes de la ferritine | | |
|---|---|---|---|
| Caractéristiques des patients | Basse (<15µg/L) | Sub normale (>15µg/L) | Normale (>34µg/L) |
| Nombre | 11 | 22 | 10 |
| Age | 9.1±2.5 | 9.6±2.1 | 8.7±2.2 |
| Sexe (M/F) | 8/3 | 18/4 | 10/0 |
| Score de Conners parental | | | |
| - Total | 66±13* | 63±11* | 52±14* |
| - Score "hyperactivité" | 19±4 | 19±4 | 17±4 |
| - score "trouble cognitif" | 10±4 | 9±4 | 8±5 |
| - score "trouble oppositionnel" | 8±3 | 9±2 | 7±2 |
| Fer sérique (µg/100 ml) | 86±27 | 81±40 | 92±44 |
| Ferritine µg/L) | 13±2 | 23±5 | 43±11 |

Les données sont indiquées en moyenne ± déviation standard
* p<0.015, différence statistiquement significative par rapport aux enfants avec une ferritinémie normale.

L'inventeur a mis en évidence qu'une férritinémie basse correspond à des réserves en fer basses chez les enfants souffrant de TDAH. Le point de rupture de la carence en fer chez l'enfant (entre 5 et 12 ans) est un taux de ferritine sérique supérieur à 15µg/L, et touche 3 % d'entre eux. En revanche, dans notre étude, 23% des TDAH ont révélé des carences en fer sévères sans anémie. De plus, il apparaît que les valeurs de la ferritine étaient inversement corrélées à la sévérité des symptômes exprimée par le questionnaire de Conners Parents. Ceci laisse supposer qu'il existe un rapport entre les réserves en fer (ferritine) et les symptômes du TDAH.

Il semble donc important d'instaurer le contrôle des réserves en fer de l'organisme (ferritinémie) de façon systématique en consultation spécialisée du TDAH. La recherche d'une carence martiale dans le TDAH et sa prise en charge thérapeutique devraient en effet précéder l'administration d'un psycho-stimulant.

## Revendications

1. Utilisation du fer pour la préparation d'un médicament destiné au traitement préventif et/ou curatif du trouble du déficit de l'attention/hyperactivité (TDAH) ou d'au moins un de ces symptômes, chez un patient nécessitant un tel traitement.

2. Utilisation selon la revendication 1 **caractérisée en ce que** le dit patient est choisi parmi un nouveau-né, un enfant, un adolescent, un adulte.

3. Utilisation selon la revendication 2, **caractérisée en ce que** ledit patient est un enfant présentant une carence martiale isolée mais non anémié.

4. Utilisation selon l'une quelconque des revendications 1 à 3 **caractérisée en ce que** le fer est sous la forme d'un atome de fer, de sel de fer ou de fer organique, pharmaceutiquement acceptables.

5. Utilisation selon la revendication 4 **caractérisée en ce que** le sel de fer pharmaceutiquement acceptable est sélectionné parmi les sels ferreux et les sels ferriques, de préférence parmi l'ammonium citrate ferrique, le pyrophosphate ferrique, la ferritine, le ferrocholinate, l'ascorbate ferreux, l'aspartate ferreux, le chlorure ferreux, le sulfate ferreux, le tartrate ferreux, le fumarate ferreux, le gluconate ferreux, le gluceptate ferreux, le sulfate de glycine ferreux, le lactate ferreux, l'oxalate ferreux, le succinate ferreux .

6. Utilisation selon la revendication 5 **caractérisé en ce que** le sel de fer est le sulfate ferreux.

7. Utilisation selon la revendication 4 **caractérisée en ce que** le fer pharmaceutiquement acceptable est sous la forme de fer dextran, de fer sucrose, de fer polymaltose, de fer sorbitol.

8. Utilisation selon la revendication 4 **caractérisée en ce que** le fer organique pharmaceutiquement acceptable est sous la forme de bi-glycinate de fer, de glycinate de fer ou de fer protéine succinylate.

9. Utilisation selon l'une quelconques des revendications précédentes **caractérisée en ce que** le médicament est formulé pour permettre l'administration du fer par voie orale, anale, parentérale, intra-musculaire ou intraveineuse.

10. Utilisation selon les revendications 1 à 9 **caractérisée en ce que** les symptômes sont sélectionnés parmi l'inattention, l'impulsivité, l'impatience, l'hyperactivité motrice diurne ou nocturne, l'insomnie, le syndrome des jambes sans repos.

11. Utilisation selon l'une quelconque des revendications 1 à 10 en association avec au moins un composé sélectionné parmi les psycho-stimulants, notamment les inhibiteurs de la recapture de la dopamine et/ou de la noradrénaline comme produit de combinaison pour une utilisation simultanée, séparée ou échelonnée dans le temps.

12. Utilisation selon la revendication 11 **caractérisée en ce que** le dit composé est choisi parmi le methylphénidate, le modafinil, l'atomoxétine, et les amphétamines, notamment la d-amphétamine, la déxédrine, la dexamphétamine, la L-Dopa, la Dopamine, les agonistes de la L-dopa, les agonistes de la dopamine, la rotaline.

13. Utilisation du fer en association avec au moins un composé sélectionné parmi les psycho-stimulants, notamment les inhibiteurs de la recapture de la dopamine et/ou de la noradrénaline, comme produit de combinaison pour une utilisation simultanée, séparée ou échelonnée dans le temps, pour la préparation d'un médicament destiné au traitement préventif et/ou curatif d'une pathologie sélectionnée parmi l'hyperactivité motrice nocturne et/ou diurne et le syndrome des jambes sans repos.

14. Utilisation selon les revendications 1 à 13 **caractérisée en ce que** la posologie martiale correspond à une prise journalière de sulfate ferreux comprise entre 0.1 mg et 10 g, de préférence comprise entre 100 mg et 2 g par jour, de préférence environ 500 mg, en une ou plusieurs prises.

15. Utilisation selon l'une quelconque des revendications 1 à 14 **caractérisée en ce que** le dit patient est affecté d'une carence en ferritine, la dite concentration sérique en ferritine du dit patient étant inférieure à 50 µg/litre, inférieure à environ 40 µg/1, inférieure à environ 35 µg/1, inférieure à environ 30 µg/1, inférieure à environ 20 µg/l,, inférieure à environ 15 µg/1, inférieure à environ 10 µg/1, inférieure à environ 5 µg/1.

16. Utilisation selon la revendication 15 **caractérisée en ce que** le dit patient présente en outre une concentration sérique normale de récepteurs solubles à la transferrine.

17. Utilisation selon l'une quelconque des revendications précédentes pour le traitement préventif de patient nouveau-né, adolescent, jeune adulte amené à développer à l'age adulte une pathologie neuro-dégénérative **caractérisée en ce que** le dit patient nouveau-né, adolescent, jeune adulte présente au moins les symptômes suivants :
- une carence en ferritine, de sorte que la concentration sérique en ferritine est inférieure à 50 µg/1,
- une concentration sérique normale des récepteurs solubles à transferrine
- un trouble du déficit de l'attention/hyperactivité, ou au moins un de ces symptômes.

18. Utilisation selon la revendication 17 **caractérisée en ce que** la dite pathologie neurodégénérative est la maladie de Parkinson.

19. Procédé in vitro de pronostic et/ou diagnostic du trouble du déficit de l'attention/ hyperactivité comprenant l'étape d'évaluer quantitativement chez un patient suspecté d'être affecté par le dit trouble, la concentration sérique de ferritine et la concentration en récepteur soluble de la transférine, de sorte qu'une concentration en ferritine sérique inférieure à 50 µg/l et une concentration normale physiologique des récepteurs solubles à la transferrine indique que le patient est ou sera affecté du dit trouble.

20. Utilisation de la ferritine et/ou des récepteurs solubles à la transferrine comme marqueur de sévérité du TDAH.

21. Composition pharmaceutique comprenant des excipients pharmaceutiquement acceptables, du fer ou un de ses sels pharmaceutiquement acceptables, et au moins un psychostimulant, de préférence la ritaline, pour le traitement préventif et/ou curatif du TDAH ou l'un de ses symptômes.
